# FASCICULE DE BREVET EUROPEEN

(11) **EP 4 034 679 B1**
(45) Date de publication et mention de la délivrance du brevet: **12.11.2025**
(21) Numéro de dépôt: 20790378.2
(22) Date de dépôt: 25.09.2020
(51) Int. Cl.: C12Q 1/6883

(54) **PROCÉDÉ POUR DÉTERMINER LE RISQUE DE SURVENUE D'UNE INFECTION ASSOCIÉE AUX SOINS CHEZ UN PATIENT**
VERFAHREN ZUR BESTIMMUNG DES RISIKOS DES AUFTRETENS EINER PFLEGE-ASSOZIIERTEN INFEKTION BEI EINEM PATIENTEN
METHOD FOR DETERMINING THE RISK OF INCIDENCE OF A CARE-ASSOCIATED INFECTION IN A PATIENT

(30) Priorité: 27.09.2019 FR 1910723
(43) Date de publication de la demande: 03.08.2022
(73) Titulaire: bioMérieux, 69280 Marcy l'Etoile (FR); Bioaster, 69007 Lyon (FR)
(72) Inventeur: VACHOT, Laurence, 38600 Fontaine (FR); MALLET, François, 69100 Villeurbanne (FR); MONNERET, Guillaume, 69008 Lyon (FR); MOUCADEL, Virginie, 38360 Sassenage (FR); PACHOT, Alexandre, 01400 Sulignat (FR); PERONNET, Estelle, 69009 Lyon (FR); RIMMELÉ, Thomas, 69007 Lyon (FR); TEXTORIS, Julien, 69100 Villeurbanne (FR); VENET, Fabienne, 69008 Lyon (FR)
(74) Mandataire: Germain Maureau
(86) Numéro de dépôt international: PCT/FR2020/051673
(87) Numéro de publication internationale: WO 2021/058919

(56) Documents cités:
- EP-A1- 2 379 752
- EP-B1- 2 379 752
- WO-A1-2018/140256
- FR-A1- 2 941 239
- FR-A1- 2 941 240
- US-A1- 2019 010 546
- MICHAIL S. LIONAKIS ET AL: "CX3CR1-dependent renal macrophage survival promotes Candida control and host survival", THE JOURNAL OF CLINICAL INVESTIGATION, vol. 123, no. 12, 1 November 2013 (2013-11-01), GB, pages 5035 - 5051, XP055694579, ISSN: 0021-9738, DOI: 10.1172/JCI71307
- TIMOTHY E. SWEENEY ET AL: "A community approach to mortality prediction in sepsis via gene expression analysis", NATURE COMMUNICATIONS, vol. 9, no. 1, 15 February 2018 (2018-02-15), XP055653297, DOI: 10.1038/s41467-018-03078-2
- KOEN VENKEN ET AL: "Natural naive CD4+CD25+CD127low regulatory T cell (Treg) development and function are disturbed in multiple sclerosis patients: recovery of memory Treg homeostasis during disease progression.", THE JOURNAL OF IMMUNOLOGY, vol. 180, no. 9, 1 May 2008 (2008-05-01), pages 6411 - 6420, XP055062918, ISSN: 0022-1767, DOI: 10.4049/jimmunol.180.9.6411
- LINA YI ET AL: "Gene expression profiling of CD4+ T cells in treatment-naive HIV, HCV mono- or co-infected Chinese", VIROLOGY JOURNAL, BIOMED CENTRAL, LONDON, GB, vol. 11, no. 1, 13 February 2014 (2014-02-13), pages 27, XP021178857, ISSN: 1743-422X, DOI: 10.1186/1743-422X-11-27
- WANG JINGWEN ET AL: "Distinctively Expressed Cytokines by Three Different Inflammation Cells and Their Interaction with Keratinocytes in Wound Healing", INFLAMMATION, PLENUM PRESS, NEW YORK, NY, US, vol. 40, no. 6, 2 September 2017 (2017-09-02), pages 2151 - 2162, XP036348312, ISSN: 0360-3997, [retrieved on 20170902], DOI: 10.1007/S10753-017-0655-9

## Description

La présente invention concerne un procédé *in vitro* ou *ex vivo* pour déterminer le risque de survenue d'une infection associée aux soins chez un patient, comprenant une étape de mesure de l'expression de CX3CR1, dans un échantillon biologique dudit patient.

Le développement d'infections associées aux soins est une complication importante liée aux soins médicaux, en particulier dans les structures de soins médicalisées comme les hôpitaux (où l'on parlera plus précisément d'infections nosocomiales). Il a été montré que les infections nosocomiales en unités de soins intensifs, qui surviennent chez 20 à 40% des patients, étaient associées à une morbidité et une mortalité accrues, une durée plus longue du besoin en soins de suppléance aux défaillances d'organe(s), une durée de séjour plus longue à l'hôpital, des coûts de santé plus importants, et une utilisation plus importante d'antibiotiques, contribuant à la résistance antimicrobienne. L'occurrence des infections associées aux soins est particulièrement exacerbée depuis quelques années, de par l'augmentation des pathogènes multirésistants. L'Organisation Mondiale de la Santé (OMS) estime à environ 5 millions le nombre d'infections nosocomiales dans les hôpitaux en Europe, conduisant à environ 50 000 morts et un surcoût annuel de 13 à 24 milliards d'euros. De nombreux facteurs influent sur la survenue et le développement des infections associées aux soins, comme l'état général de santé du patient, mais également des facteurs liés à la prise en charge du patient (e.g. l'administration d'antibiotiques et/ou l'utilisation de dispositifs médicaux invasifs), des facteurs liés à l'environnement hospitalier (e.g. le ratio du nombre d'infirmières par rapport au nombre de patients), et l'utilisation variable des techniques aseptiques par le personnel hospitalier. Des recommandations ont été publiées, et la mise en place de programmes de contrôle des infections a été encouragée, en particulier par le *U.S. Department of Health and Human Services,* le Centre européen pour la prévention et le contrôle des maladies, l'OMS et les agences nationales, pour lesquels la prévention et la réduction des infections associées aux soins sont devenues une priorité majeure. Il a été montré que les programmes de contrôle des infections associées aux soins se révèlent particulièrement efficaces pour réduire les infections sévères. Cependant, il a été estimé qu'un maximum de 65 à 70% des cas d'infections du sang et des voies urinaires, liées à la pose de cathéters, et de 55% des cas de pneumonie associées à la ventilation mécanique et d'infections au niveau du site chirurgical, pourraient être évités. Par ailleurs, l'observance et l'application des procédures selon les recommandations peuvent être compliquées dans certains hôpitaux, particulièrement dans les pays à faibles ou moyens revenus. L'identification précoce des patients à risque de développer une infection associée aux soins serait une étape-clé dans la prévention de ces infections et la prise en charge de ces patients. D'après certaines modélisations, un biomarqueur qui réduirait le temps d'identification des infections associées aux soins chez une population à haut risque, permettrait de réduire la mortalité chez ces patients, avec un bon rapport coût/efficacité. Cependant, il n'existe actuellement pas en clinique de test de diagnostic *in vitro* permettant d'identifier les patients à haut risque de contracter une infection associée aux soins.

Or, il a été découvert que, de façon tout-à-fait surprenante, la mesure de l'expression du gène CX3CR1, qui code pour le récepteur à la fractalkine (ou CX3CL1), permettait de déterminer le risque de survenue d'une infection associée aux soins chez un patient. Les patients ayant un risque élevé de contracter une infection associée aux soins pourraient avantageusement bénéficier d'un traitement immunitaire immunostimulant ou d'une prise en charge individualisée. Dans la littérature, une diminution de l'expression de CX3CR1 a déjà été montrée comme étant associée à une augmentation de la mortalité, notamment chez des patients en état septique, et plus particulièrement en choc septique (Pachot et al (2008), J Immunol 180 : 6421-6429), mais l'utilité de la mesure de l'expression de CX3CR1 pour la prédiction de survenue d'infections associées aux soins n'avait encore jamais été montrée ou suggérée.

Ainsi, la présente invention a pour objet un procédé *in vitro* ou *ex vivo* pour déterminer le risque de survenue d'une infection nosocomiale chez un patient au sein d'un établissement de santé, comprenant une étape de mesure de l'expression de CX3CR1 (localisation chromosomique du gène selon le GRCh38/hg38 : chr3:39,263,494-39,281,735), dans un échantillon biologique dudit patient.

Dans le cadre de la présente invention :
- Le terme « patient » désigne un individu (être humain), qui est entré en contact avec un professionnel de santé, tel qu'un médecin (par exemple, un médecin généraliste) ou une structure médicale ou un établissement de santé (par exemple, un hôpital, et plus particulièrement le service des urgences, le service de réanimation, une unité de soins intensifs ou une unité de soins continus, ou une structure médicalisée pour personnes âgées, de type EHPAD). Le patient peut par exemple être une personne âgée, dans le cadre d'un protocole de vaccination (notamment en EHPAD ou encore chez un médecin généraliste) ;
- Une infection est dite « associée aux soins », si elle survient au cours ou au décours d'une prise en charge (diagnostique, thérapeutique, palliative, préventive, éducative ou opératoire) d'un patient par un professionnel de santé, et si elle n'était ni présente, ni en incubation au début de la prise en charge. Les infections associées aux soins (IAS) comprennent les infections contractées au sein d'un établissement de santé (dites infections nosocomiales) mais aussi lors de soins délivrés hors de ce cadre. Lorsque l'état infectieux au début de la prise en charge n'est pas connu précisément, un délai d'au moins 48 heures ou un délai supérieur à la période d'incubation est couramment accepté pour définir une IAS. Pour les infections du site opératoire, on considère habituellement comme associées aux soins les infections survenant dans les 30 jours suivant l'intervention ou, s'il y a mise en place d'un implant, d'une prothèse ou d'un matériel prothétique dans l'année qui suit l'intervention. L'infection peut être d'origine bactérienne, fongique ou encore virale. Il peut s'agir également de la réactivation de virus latents potentiellement pathogènes, tels TTV ou les virus d'herpes, par exemple le CMV ;
- Par « échantillon biologique », on se réfère à tout échantillon provenant d'un patient, et pouvant être de différentes natures, comme le sang ou ses dérivés, les expectorations, l'urine, les selles, la peau, le liquide céphalo-rachidien, le liquide de lavage broncho-alvéolaire, la salive, les sécrétions gastriques, le sperme, le liquide séminal, les larmes, la moelle épinière, ganglion du nerf trijumeau, tissu adipeux, tissu lymphoïde, tissu placentaire, tissu du tractus gastro-intestinal, tissu du tractus génital, tissu du système nerveux central. En particulier, cet échantillon peut être un fluide biologique, comme un échantillon de sang ou un échantillon dérivé du sang, qui peut notamment être choisi parmi le sang total (tel que collecté de la voie veineuse, c'est-à-dire contenant les cellules blanches et rouges, les plaquettes et le plasma), le plasma, le sérum, ainsi que tout(tous) type(s) de cellules extraites à partir du sang, telles que les cellules mononuclées sanguines périphériques (ou PBMC, contenant les lymphocytes (B, T et cellules NK), les cellules dendritiques et les monocytes), des sous-populations de cellules B, des monocytes purifiés, ou des neutrophiles.

De préférence, dans le procédé tel que décrit précédemment :
- le patient est un patient au sein d'un établissement de santé, de préférence au sein d'un hôpital, de préférence encore au sein du service des urgences, du service de réanimation, en unité de soins intensifs ou en unité de soins continus ; de manière particulièrement préférée, le patient est un patient en état septique (plus particulièrement, en choc septique), un patient atteint de brûlures (plus particulièrement, de brûlures graves), un patient atteint de traumatismes (plus particulièrement, de traumatismes graves), ou un patient opéré par chirurgie (plus particulièrement, une chirurgie lourde) ; et
- le procédé permet de déterminer le risque de survenue d'une infection nosocomiale chez ledit patient.

Dans le cas d'un patient en état septique (déjà atteint d'une première infection), le procédé selon l'invention permet de déterminer le risque de survenue d'une infection secondaire.

Par patient en état septique (ou patient atteint de sepsis), on entend un patient présentant au moins une défaillance d'organe menaçant le pronostic vital et causé par une réponse inappropriée de l'hôte à une infection. Par choc septique, on entend un sous-type de sepsis, dans lequel une hypotension persiste, malgré un remplissage vasculaire adéquat.

De préférence, le procédé selon l'invention, tel que décrit précédemment, dans tous ses modes de réalisation, permet de déterminer le risque de survenue d'une infection associée aux soins chez un patient :
- dans les 15 jours à partir du jour de l'agression immuno-inflammatoire (*i.e.* le traumatisme pour les patients atteints de traumatismes, la brûlure pour les patients atteints de brûlures, la chirurgie pour les patients opérés par chirurgie ou le diagnostic de sepsis pour les patients en état septique), c'est-à-dire au cours du 1^{er}, du 2^{ème}, du 3^{ème}, du 4^{ème}, du 5^{ème}, du 6^{ème}, du 7^{ème}, du 8^{ème}, du 9^{ème}, du 10^{ème}, du 11^{ème}, du 12^{ème}, du 13^{ème}, du 14^{ème} ou du 15^{ème} jour à partir de l'agression immuno-inflammatoire (le 1^{er} jour correspondant ici au jour de survenue de l'agression immuno-inflammatoire) ; le prélèvement de l'échantillon biologique pouvant en particulier avoir été effectué au cours du 1^{er}, du 2^{ème}, du 3^{ème}, du 4^{ème}, du 5^{ème}, du 6^{ème}, du 7^{ème}, du 8^{ème}, du 9^{ème}, du 10^{ème}, du 11^{ème}, du 12^{ème}, du 13^{ème}, du 14^{ème} ou du 15^{ème} jour à partir de l'agression immuno-inflammatoire, de préférence au cours du 1^{er}, du 2^{ème}, du 3^{ème}, du 4^{ème}, du 5^{ème}, du 6^{ème} ou du 7^{ème} jour à partir de l'agression immuno-inflammatoire, de préférence encore au cours du 3^{ème}, du 4^{ème}, du 5^{ème}, du 6^{ème} ou du 7^{ème} jour à partir de l'agression immuno-inflammatoire ; et/ou
- dans les 7 jours, dans les 6 jours, dans les 5 jours ou dans les 4 jours suivant le jour où le prélèvement de l'échantillon biologique a été effectué (quel que soit le jour où ce prélèvement a été effectué), c'est-à-dire au cours du 1^{er}, du 2^{ème}, du 3^{ème}, du 4^{ème}, du 5^{ème}, du 6^{ème} ou du 7^{ème} jour suivant le jour où le prélèvement de l'échantillon biologique a été effectué (le 1^{er} jour correspondant ici au lendemain du jour où le prélèvement de l'échantillon biologique a été effectué).

De préférence, dans le procédé tel que décrit précédemment, dans tous ses modes de réalisation, l'échantillon biologique est un échantillon de sang, de préférence un échantillon de sang total ou un échantillon dérivé du sang (e.g. des PBMC, qui peuvent être obtenues par la méthode Ficoll, bien connue de l'homme du métier, ou des monocytes purifiés).

De préférence, le procédé tel que décrit précédemment, dans tous ses modes de réalisation, comprend en outre une étape de mesure, dans l'échantillon biologique du patient, de l'expression d'un autre gène d'intérêt, sélectionné dans la liste constituée par : ADGRE3, BTLA, CD3D, CD74, CD274 (également connu sous le nom PD-L1), CTLA4 (également connu sous le nom CD152), HP, ICOS, IFNG, IL1RN, IL6, IL7R (également connu sous le nom CD127), IL10, IL15, MDC1, PDCD1 (également connu sous les noms PD-1 et CD279), S100A9, TDRD9 et ZAP70 ; de préférence encore, l'autre gène d'intérêt est sélectionné dans la liste constituée par : BTLA, CD3D, CD74, CD274, CTLA4, HP, ICOS, IFNG, IL1RN, IL7R, IL10, IL15, PDCD1 et S100A9.

**Tableau 1. Localisation chromosomique des gènes dont l'expression peut être mesurée en combinaison avec la mesure de l'expression de CX3CR1**

| **Biomarqueur (gène)** | **Localisation chromosomique (GRCh38/hg38)** |
|---|---|
| ADGRE3 | chr19:14,619,117-14,690,027 |
| BTLA | chr3:112,463,966-112,499,702 |
| CD3D | chr11:118,338,954-118,342,744 |
| CD74 | chr5: 150,400,041-150,412,936 |
| CD274 | chr9:5,450,381-5,470,567 |
| CTLA4 | chr2:203,867,771-203,873,965 |
| HP | chr16:72,054,592-72,061,056 |
| ICOS | chr2:203,936,731-203,961,579 |
| IFNG | chr12:68,154,768-68,159,741 |
| IL1RN | chr2:113,099,365-113,134,016 |
| IL6 | chr7:22,725,442-22,732,002 |
| IL7R | chr5:35,852,695-35,879,603 |
| IL10 | chr1:206,767,602-206,774,607 |
| IL15 | chr4:141,636,583-141,733,987 |
| MDC1 | chr6:30,699,807-30,717,966 |
| PDCD1 | chr2:241,849,881-241,858,908 |
| S100A9 | chr1:153,357,854-153,361,027 |
| TDRD9 | chr14:103,928,438-104,052,667 |
| ZAP70 | chr2:97,713,560-97,744,327 |

La mesure de l'expression (ou du niveau d'expression) d'un gène consiste à quantifier au moins un produit d'expression du gène. Le produit d'expression d'un gène, au sens de la présente invention, est toute molécule biologique issue de l'expression dudit gène.

Plus particulièrement, le produit d'expression du gène peut être un transcrit ARN. Par « transcrit », on entend les ARN, et en particulier les ARN messagers (ARNm), issus de la transcription du gène. Plus précisément, les transcrits sont les ARN produits par la transcription d'un gène suivi des modifications post-transcriptionnelles des formes pré-ARN. Dans le cadre de la présente invention, la mesure du niveau d'expression d'un ou plusieurs transcrits ARN du même gène peut être effectuée. Ainsi, de préférence, dans le procédé tel que décrit précédemment, dans tous ses modes de réalisation, l'expression du(des) gène(s) (*i.e.* l'expression de CX3CR1, et éventuellement d'un autre gène d'intérêt de la liste indiquée précédemment) est mesurée au niveau transcrit ARN ou ARNm. Dans le cas d'un transcrit ARNm, la détection peut être réalisée par une méthode directe, par tout procédé connu de l'homme du métier permettant de déterminer la présence dudit transcrit dans l'échantillon, ou par détection indirecte du transcrit après transformation de ce dernier en ADN, ou après amplification dudit transcrit ou après amplification de l'ADN obtenu après transformation dudit transcrit en ADN. De nombreuses méthodes existent pour la détection des acides nucléiques (voir par exemple Kricka et al., Clinical Chemistry, 1999, n° 45(4), p.453-458 ; Relier G. H. et al., DNA Probes, 2nd Ed., Stockton Press, 1993, sections 5 et 6, p.173-249). L'expression des gènes peut notamment être mesurée par *Reverse Transcription-Polymerase Chain Reaction* ou RT-PCR, de préférence par RT-PCR quantitative ou RT-qPCR (par exemple en utilisant la technologie FilmArray^{®}), par séquençage (de préférence par séquençage haut débit) ou par des techniques d'hybridation (par exemple avec des micropuces d'hybridation ou par des techniques du type NanoString^{®} nCounter^{®}).

Le produit d'expression du gène peut également être une protéine et/ou un polypeptide qui est le produit de la traduction d'au moins un des transcrits dudit gène. Ainsi, dans le procédé tel que décrit précédemment, l'expression du(des) gène(s) peut également être mesurée au niveau protéine. Toutes les isoformes du(des) protéine(s), produit(s) d'expression du(des) gène(s), peuvent être mesurées, seules ou en combinaison, en tant que marqueur(s) pour déterminer le risque de survenue d'une infection associée aux soins chez un patient. La mesure de l'expression de gène(s) au niveau protéique dans un échantillon biologique peut se faire selon les techniques largement connues de l'homme du métier pour déterminer la quantité, ou dose, d'un ou plusieurs analytes dans un échantillon biologique. A titre d'exemples, on peut citer les dosages par immunoessais, tels qu'ELISA (*Enzyme Linked Immuno Sorbent Assay*), ELFA (*Enzyme Linked Fluorescent Assay*) et RIA (*Radio Immuno Assay*), et les dosages par spectrométrie de masse.

La mesure du niveau d'expression d'un gène permet de déterminer la quantité d'un ou plusieurs transcrits (ou d'une ou plusieurs protéines) présents dans l'échantillon biologique ou d'en donner une valeur dérivée. Une valeur dérivée de la quantité peut par exemple être la concentration absolue, calculée grâce à une courbe de calibration obtenue à partir de dilutions successives d'une solution d'amplicons (ou de protéines ou polypeptides) de concentration connue. Elle peut également correspondre à la valeur de la quantité normalisée et calibrée, comme le CNRQ (Calibrated Normalized Relative Quantity, (Hellemans et al (2007), Genome biology 8(2):R19), qui intègre les valeurs d'un échantillon de référence (ou d'un calibrateur) et d'un ou plusieurs gènes de ménage (appelés également gènes de référence). A titre d'exemples de gènes de ménage, on peut citer les gènes DECR1, HPRT1, PPIB, RPLP0, PPIA, GLYR1, RANBP3, 18S, GAPDH et ACTB.

Ainsi, de préférence, dans le procédé tel que décrit précédemment, dans tous ses modes de réalisation, l'expression du(des) gène(s) d'intérêt est normalisée par rapport à l'expression d'un ou plusieurs gènes de ménage (ou gènes de référence), comme cela est connu de l'homme du métier ; de préférence encore en utilisant un ou plusieurs des gènes de ménage suivants : DECR1 (localisation chromosomique du gène selon le GRCh38/hg38 : chr8:90,001,352-90,053,633), HPRT1 (localisation chromosomique du gène selon le GRCh38/hg38 : chrX:134,452,842-134,520,513) et PPIB (localisation chromosomique du gène selon le GRCh38/hg38 : chr15:64,155,812-64,163,205).

De préférence, dans le procédé tel que décrit précédemment, dans tous ses modes de réalisation, l'expression du(des) gènes d'intérêt (de préférence, l'expression normalisée) dans l'échantillon biologique du patient est comparée à une valeur de référence ou à l'expression du(des) mêmes gènes d'intérêt (de préférence, l'expression normalisée) dans un échantillon biologique de référence (ces données étant utilisées pour le calcul du CNRQ, comme mentionné plus haut). L'échantillon de référence peut être par exemple un échantillon provenant d'un volontaire (individu sain), d'un patient, ou un mélange d'échantillons de plusieurs volontaires (d'une part) ou de plusieurs patients (d'autre part). L'échantillon de référence peut aussi être un échantillon prélevé d'un volontaire (ou un mélange d'échantillons prélevés sur plusieurs volontaires) puis traité *ex vivo* par un agent stimulant du système immunitaire (tel que le LPS ou lipopolysaccharide). L'échantillon de référence peut également être un mélange d'échantillon(s) non traités et d'échantillons traité(s) *ex vivo* par un agent stimulant du système immunitaire.

De préférence, le procédé pour déterminer le risque de survenue d'une infection associée aux soins, tel que décrit précédemment, dans tous ses modes de réalisation, comprend également une étape de gestion des soins de santé pour réduire le risque de survenue d'une infection associée aux soins. Un patient identifié comme étant à risque accru de survenue d'une infection associée aux soins peut avoir une gestion des soins de santé adaptée dans le but de réduire le risque de survenue d'une infection associée aux soins et, par exemple, afin de réduire le risque de développer un sepsis, un choc septique ou encore le risque de décès. A titre d'exemples de gestion des soins, on peut citer un traitement immunomodulateur adapté au patient ou encore un traitement antibiotique prophylactique, les deux traitements pouvant être associés et/ou orienter vers un service de soins continus ou de réanimation afin de réduire le risque de survenue d'une infection associée aux soins, par exemple réduire le risque de développer un sepsis, un choc septique ou même le risque de décès dans les jours qui suivent la mesure de l'expression du ou des biomarqueur(s). De manière préférée, le traitement immunomodulateur est un traitement immunostimulant, s'il est déterminé que l'individu a un statut d'immunosuppression, ou un traitement anti-inflammatoire, s'il est déterminé que l'individu a un statut d'inflammation. Parmi les traitements immunostimulants qui peuvent être sélectionnés, on peut citer à titre d'exemples le groupe des interleukines, en particulier IL-7, IL-15 ou IL-3, des facteurs de croissance, en particulier le GM-CSF, des interférons, en particulier IFNγ, des Toll agonistes, des anticorps, en particulier des anticorps anti-PD1, anti-PDL1, anti-LAG3, anti-TIM3, anti-IL-10 ou anti-CTLA4, des transferrines et des molécules inhibitrices de l'apoptose, FLT3L, Thymosin α1, des antagonistes adrénergiques. Parmi les traitements anti-inflammatoires, on peut citer notamment le groupe des glucocorticoïdes, des agents cytostatiques, des molécules agissant sur les immunophilines et les cytokines, des molécules bloquant le récepteur à l'IL-1 et des traitements anti-TNF. Des exemples de traitements antibiotiques prophylactiques appropriés pour prévenir la pneumonie sont décrits en particulier dans les Annales Françaises d'Anesthésie et de Réanimation (30 ; 2011 ; 168-190). Inversement, un patient ne présentant pas de risque de survenue d'une infection associée aux soins pourra être orienté rapidement vers un service d'hospitalisation de jour, par exemple un service d'infectiologie, plutôt que de rester dans un service avec un monitoring rapproché dont il n'aura pas besoin.

Sans faire partie de l'invention, la demande décrit un kit comprenant des moyens d'amplification et/ou des moyens de détection de l'expression (de préférence des amorces et/ou des sondes, ou des anticorps) de CX3CR1 et d'un autre gène, sélectionné dans la liste constituée par : ADGRE3, BTLA, CD3D, CD74, CD274, CTLA4, HP, ICOS, IFNG, IL1RN, IL6, IL7R, IL10, IL15, MDC1, PDCD1, S100A9, TDRD9 et ZAP70 (de préférence, l'autre gène est sélectionné dans la liste constituée par : BTLA, CD3D, CD74, CD274, CTLA4, HP, ICOS, IFNG, IL1RN, IL7R, IL10, IL15, PDCD1 et S100A9 ou dans la liste constituée par : ADGRE3, BTLA, CD3D, CD74, CD274, CTLA4, HP, IFNG, IL1RN, IL6, IL7R, IL10, MDC1, PDCD1, S100A9, TDRD9 et ZAP70 ; de préférence encore dans la liste constituée par : BTLA, CD3D, CD74, CD274, CTLA4, HP, IFNG, IL1RN, IL7R, IL10, PDCD1 et S100A9); ledit kit étant caractérisé en ce que l'ensemble des moyens d'amplification et/ou de détection dudit kit permettent la détection et/ou l'amplification d'au plus 100, de préférence au plus 90, de préférence au plus 80, de préférence au plus 70, de préférence au plus 60, de préférence au plus 50, de préférence au plus 40, de préférence au plus 30, de préférence au plus 20, de préférence au plus 10, de préférence au plus 5 biomarqueurs, de préférence au plus 4, de préférence au plus 3, de préférence au plus 2, au total. Par « biomarqueur » (ou « marqueur »), on entend une caractéristique biologique mesurable objectivement qui représente un indicateur des processus biologiques normaux ou pathologiques ou de réponse pharmacologique à une intervention thérapeutique. Ce biomarqueur peut en particulier être détectable au niveau ARNm ou protéine. Plus particulièrement, le biomarqueur peut être un biomarqueur endogène ou loci (tel qu'un gène ou un HERV / *Human Endogenous RetroVirus,* qui se retrouvent dans le matériel chromosomique d'un individu) ou un biomarqueur exogène (tel qu'un virus).

Ainsi, ledit kit peut par exemple comprendre également des moyens d'amplification et/ou de détection d'un ou plusieurs gènes de ménage (de préférence sélectionnés dans la liste constituée par : DECR1, HPRT1 et PPIB). Le kit peut également comprendre des moyens de contrôle positif permettant de qualifier la qualité de l'extraction de l'ARN, la qualité de tout procédé d'amplification et/ou d'hybridation.

On entend par « amorce » ou « amorce d'amplification », un fragment nucléotidique pouvant être constitué de 5 à 100 nucléotides, de préférence de 15 à 30 nucléotides, et possédant une spécificité d'hybridation avec une séquence nucléotidique cible, dans des conditions déterminées pour l'initiation d'une polymérisation enzymatique, par exemple dans une réaction d'amplification enzymatique de la séquence nucléotidique cible. Généralement, on utilise des « couples d'amorces », constitués de deux amorces. Lorsque l'on souhaite réaliser l'amplication de plusieurs biomarqueurs (e.g des gènes) différents, plusieurs couples d'amorces différents sont de préférence utilisés, ayant préférentiellement chacun une capacité à s'hybrider spécifiquement avec un biomarqueur différent.

On entend par « sonde » ou « sonde d'hybridation », un fragment nucléotidique constitué typiquement de 5 à 100 nucléotides, de préférence de 15 à 90 nucléotides, de manière encore plus préférée de 15 à 35 nucléotides, possédant une spécificité d'hybridation dans des conditions déterminées pour former un complexe d'hybridation avec une séquence nucléotidique cible. La sonde comporte également un rapporteur (tel qu'un fluorophore, une enzyme ou tout autre système de détection), qui va permettre la détection de la séquence nucléotidique cible. Dans la présente invention, la séquence nucléotidique cible peut être une séquence nucléotidique comprise dans un ARN messager (ARNm) ou une séquence nucléotidique comprise dans un ADN complémentaire (ADNc) obtenu par transcription inverse dudit ARNm. Lorsque l'on souhaite cibler plusieurs biomarqueurs (e.g. des gènes) différents, plusieurs sondes différentes sont de préférence utilisées, ayant préférentiellement chacune une capacité à s'hybrider spécifiquement avec un biomarqueur différent.

Par « hybridation », on entend le processus au cours duquel, dans des conditions appropriées, deux fragments nucléotidiques, tels que par exemple une sonde d'hybridation et un fragment nucléotidique cible, ayant des séquences suffisamment complémentaires, sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Par « réaction d'amplification enzymatique », on entend un processus générant de multiples copies d'un fragment nucléotidique cible, par l'action d'au moins une enzyme. De telles réactions d'amplification sont bien connues de l'homme du métier et on peut citer notamment les techniques suivantes : PCR (*Polymerase Chain Reaction*), LCR (*Ligase Chain Reaction*), RCR (*Repair Chain Reaction*), 3SR (*Self Sustained Séquence Replication*) avec la demande de brevet WO-A-90/06995, NASBA (*Nucleic Acid Sequence-Based Amplification*), TMA (*Transcription Mediated Amplification*) avec le brevet US-A-5,399,491, et LAMP (*Loop mediated isothermal amplification*) avec le brevet US6410278. Lorsque la réaction d'amplification enzymatique est une PCR, on parlera plus particulièrement de RT-PCR (RT pour « *reverse transcription* »), lorsque l'étape d'amplification est précédée d'une étape de réverse-transcription d'ARN messager (ARNm) en ADN complémentaire (ADNc), et de qPCR ou RT-qPCR lorsque la PCR est quantitative.

L'invention a également pour objet l'utilisation :
- de moyens d'amplification et/ou de moyens de détection de l'expression (de préférence des amorces et/ou des sondes, ou des anticorps) de CX3CR1,
pour déterminer le risque de survenue d'une infection associée aux soins, de préférence d'une infection nosocomiale, chez un patient, de préférence un patient au sein d'un établissement de santé, de préférence encore au sein d'un hôpital, de préférence encore au sein du service des urgences, du service de réanimation, en unité de soins intensifs ou en unité de soins continus ; de manière particulièrement préférée, le patient est un patient en état septique (plus particulièrement en choc septique), un patient atteint de brûlures (plus particulièrement, de brûlures graves), un patient atteint de traumatismes (plus particulièrement, de traumatismes graves), ou un patient opéré par chirurgie (plus particulièrement, une chirurgie lourde).

La présente invention est illustrée de manière non limitative par les exemples suivants.

### Exemple 1 : La mesure de l'expression de CX3CR1 permet de prédire le risque de survenue d'une infection associée aux soins chez un patient

### Matériels et Méthodes

Une étude clinique observationnelle prospective, longitudinale et monocentrique a été réalisée à l'Hôpital Edouard Herriot (Lyon, France). Le design de cette étude clinique a été publié dans Rol et al (2017), BMJ Open 7(6) : e015734. L'étude clinique a été approuvée par l'Agence Nationale de Sécurité du Médicament et des produits de santé (ANSM) en novembre 2015 et le Comité de Protection des Personnes Sud-Est Il en décembre 2015. Des amendements au protocole ont été effectués en juillet 2016, puis en janvier 2017. Brièvement, un total de 377 patients, en état septique (n=35) ou en choc septique (n=72), atteints de brûlures graves (n=24), de traumatismes graves (n=137) ou hospitalisés dans un service de réanimation ou une unité de soins intensifs après une chirurgie majeure (n=109), et 175 volontaires sains ont été inclus entre décembre 2015 et mars 2018.
- Patients en état septique/en choc septique : selon le premier protocole clinique, seuls les patients en choc septique étaient inclus, sur la base d'une suspicion d'un foyer infectieux, un début de traitement par catécholamines dans les 48h suivant l'admission en réanimation et d'un traitement au catécholamines (noradrénaline) > 0.25 µg/kg/min pendant au moins 2 heures. Ensuite, les critères d'éligibilité ont été modifiés en août 2016, suite à la publication d'une nouvelle définition du choc septique, Sepsis 3 (Singer et al (2016), JAMA 810-801:(8)315). Les patients en choc septique étaient donc inclus sur la base d'une suspicion d'un foyer infectieux, un début de traitement par catécholamines dans les 48h suivant l'admission en réanimation et d'une thérapie vasopressive nécessaire pour maintenir une pression artérielle ≥ 65 mm Hg et concentration en lactate > 2 mmol/L (18 mg/dL), malgré la correction d'une hypovolémie. En 2017, a été ajoutée la possibilité d'inclure des patients en état septique (selon la définition Sepsis 3), à savoir la suspicion d'un foyer infectieux et l'augmentation du score SOFA ≥ 2 points par rapport au SOFA de base dans les 48h qui suivent l'admission en réanimation. Pour cette population, le jour 1 correspond au jour du diagnostic du sepsis ou du choc septique ;
- Traumatismes graves : dans le premier protocole, seuls les patients avec un traumatisme grave ont été inclus (Injury Severity Score (ISS) ≥ 25). En août 2016, a été ajoutée la possibilité d'inclure également des traumatismes moins graves (16 < ISS < 24). Pour cette population, le jour 1 correspond au jour d'admission en en service de réanimation ou unité de soins intensifs (~jour du traumatisme) ;
- Chirurgie majeure : dans le premier protocole, seules une œsogastrectomie, une résection de la vessie de type Bricker, une duodénopancréatectomie céphalique et chirurgie de l'aorte abdominale par laparotomie ont été considérées. D'autres types de chirurgie à haut risque de complication ont été ajoutés en janvier 2017 : une pancréatectomie (totale ou caudale), des tumeurs neuroendocrines, une hépatectomie (sur le côté droit), une colectomie étendue (laparotomie), une résection abdopérinéale, une néphrectomie (laparotomie, PKD), un pontage ilio-fémoral (Scarpa). Pour cette population, le jour 1 correspond au jour de la chirurgie ;
- Brûlures graves : les patients ont été sélectionnés sur la base d'une surface totale de brûlures supérieure à 30%. Pour cette population, le jour 1 correspond au jour d'admission en service de réanimation ou unité de soins intensifs (" jour de la brûlure).

Les critères d'exclusion portaient essentiellement sur des facteurs qui auraient pu impacter le statut immunitaire et biaiser les résultats (par exemple : une neutropénie sévère, des traitements corticostéroïdes, une pathologie onco-hématologique...). Chaque événement conduisant à une suspection d'infection associée aux soins, survenant au sein de l'hôpital avant le jour 30 a été revu indépendamment par trois médecins non impliqués dans le recrutement des patients. Vingt-six pourcents des patients ont développé au moins une infection associée aux soins avant le jour 30, ou avant la sortie de l'hôpital.

Des échantillons de sang ont été collectés dans des tubes PAXgene^{®} (ref. 762165, PreAnalytiX GmbH Hombrechtikon Switzerland), une fois pour les volontaires sains, et plusieurs fois pour les patients, i.e. 3-4 fois la première semaine (aux jours 1 ou 2 : J1/2, aux jours 3 ou 4 : J3/4 et aux jours 5, 6 ou 7 : J5/7), puis 3 fois à des temps plus tardifs (autour du J14, du J28 et du J60).

Le niveau d'expression de CX3CR1 a été mesuré dans ces échantillons par RT-qPCR. L'ARN a été extrait des échantillons de sang total en utilisant le *Maxwell HT Simply RNA kit* (ref. AX2420, Promega) et la plateforme automatisée EVO (TECAN), suivant les instructions du fournisseur du kit. Ensuite, 10 ng d'ARN total ont été rétro-transcrits en ADN complémentaire (ADNc), en utilisant le master mix *Fluidigm Reverse transcription* (ref. PN100-6472 A1, Fluidigm), suivant les instructions du fournisseur. L'expression de CX3CR1 a alors été quantifiée par qPCR, en utilisant le système de PCR en temps réel Biomark HD de Fluidigm.

D'abord, une étape de préamplification du cDNA a été réalisée selon les recommandations du fournisseur en utilisant le master mix PreAmp (Ref. PN100-5876 B1, Fluidigm). Ensuite, une dilution au 1/5 des cDNA préamplifiés a été faite et les qPCR ont été réalisées sur des circuits intégrés de fluidique 192.24 (Ref. PN100-6170 C1), comme recommandé par le fournisseur. Les références des sondes et amorces utilisées pour la qPCR sont présentées dans le Tableau 2.

Les cycles seuils (ou Ct) ont ensuite été déterminés. La normalisation de l'expression de CX3CR1 en CNRQ (Quantité Relative Normalisée Calibrée ou *Calibrated Normalized Relative Quantity*) a été réalisée en utilisant la moyenne géométrique des Ct de 3 gènes de ménage (DECR1, HPRT1, PPIB) et un calibrateur (correspondant à un mélange d'échantillons traités *ex vivo* par du LPS (agent stimulant du système immunitaire) (50%) et d'échantillons non traités *ex vivo* (50%), provenant de volontaires/individus sains) dans chaque circuit intégré de fluidique 192.24, tel que décrit dans Hellemans et al (2007), Genome biology 8(2):R19.

**Tableau 2. Sondes et amorces utilisées pour la qPCR**

| **Biomarqueur (gène)** | **Type** | **Référence du fournisseur des sondes et amorces utilisées** |
|---|---|---|
| CX3CR1 | Gène d'intérêt | Hs04971470_s1 (ThermoFisher) |
| DECR1 | Gène de référence | Hs00154728_m1 (ThermoFisher) |
| HPRT1 | Gène de référence | Hs99999909_m1 (ThermoFisher) |
| PPIB | Gène de référence | Hs01018503_m1 (ThermoFisher) |

En ce qui concerne l'analyse des données, les associations entre l'expression de CX3CR1, mesurée à différents temps durant la première semaine, et la survenue d'une infection associée aux soins avant le jour 30 à partir de l'inclusion dans l'étude ont été évaluées. Les résultats ont été calculés sous la forme d*'Hazard Ratios* exprimés en distance inter-quartile avec l'intervalle de confiance à 95% associé (HR IQR). Ensuite, des régressions logistiques univariées ont été implémentées pour prédire le risque d'occurrence d'une infection associée aux soins avant le jour 15. La puissance des valeurs prédites par la régression logistique pour discriminer entre infection associée aux soins et absence d'infection associée aux soins a été quantifiée par l'aire sous la courbe (AUC) de la courbe ROC (*Receiver Operating Characteristic*), et des intervalles de confiance à 95% ont été estimés.

Ensuite, l'association entre l'expression de CX3CR1 et l'occurrence d'une infection associée aux soins a été évaluée pour différents intervalles de temps de survenue de l'infection (i.e. délais entre le prélèvement d'échantillon et la 1^{ère} survenue d'une infection). Les différents délais considérés étaient : une infection associée aux soins dans les 4 jours et dans les 7 jours après le prélèvement d'échantillon, quel que soit le moment où l'échantillon a été prélevé. Pour chaque patient ayant développé une infection associée aux soins, l'échantillon considéré correspond au plus proche prélèvement avant la survenue du premier épisode d'infection associée aux soins. Pour les patients n'ayant pas développé d'infection associée aux soins (i.e. patients contrôles), une méthode d'appariement a été utilisée pour sélectionner pour chaque cas, un patient contrôle ayant le même jour de prélèvement de l'échantillon, et des scores SOFA et Charlson proches. Finalement, un unique contrôle a été sélectionné pour chaque cas unique. Des régressions logistiques univariées ont été implémentées. La puissance des valeurs prédites par la régression logistique pour discriminer entre infection associée aux soins et absence d'infection associée aux soins a été quantifiée par l'aire sous la courbe (AUC) de la courbe ROC, et des intervalles de confiance à 95% ont été estimés.

### Résultats

Une diminution de l'expression de CX3CR1 au niveau ARNm, mesurée à J3/4 ou à J5/7 à partir de l'inclusion dans la cohorte, est associée avec un risque plus élevé d'occurrence d'une infection associée aux soins avant le J30 (J3/4: HR IQR=0.54 [0.39-0.74], p=0.0001; J5/7: HR IQR=0.57 [0.42-0.79], p=0.0006) dans la population globale des patients. Cette association était toujours significative, pour les deux temps de mesure de l'expression de CX3CR1, après ajustement avec les scores SOFA et Charlson (J3/4: HR IQR=0.61 [0.44-0.85], p=0.003; J5/7: HR IQR=0.65 [0.46-0.91], p=0.01).

Par ailleurs, les modèles de prédiction ont montré que l'expression de CX3CR1 au niveau ARNm, mesurée à J3/4 ou à J5/7 à partir de l'inclusion dans la cohorte, permettait de prédire l'occurrence d'une infection associée aux soins avant le jour 15 à partir de l'inclusion dans la cohorte (Tableau 3).

**Tableau 3. Performance (AUC et intervalle de confiance à 95%, IC) de la mesure de l'expression de CX3CR1, mesurée à J3/4 ou à J5/7 à partir de l'inclusion dans la cohorte, pour la prédiction de survenue d'une infection associée aux soins avant le jour 15 à partir de l'inclusion dans la cohorte.**

| **Jour de prélèvement de l'échantillon** | **AUC (IC)** |
|---|---|
| J3/4 | 0,677 (0,571-0,783) |
| J5/7 | 0,758 (0,655-0,86) |

Les modèles de prédiction ont également montré que l'expression de CX3CR1 au niveau ARNm, mesurée à J3/4 ou à J5/7, permettait de prédire l'occurrence d'une infection associée aux soins dans les 4 jours ou dans les 7 jours suivant le prélèvement de l'échantillon (Tableau 4).

**Tableau 4. Performance (AUC et intervalle de confiance à 95%, IC) de la mesure de l'expression de CX3CR1, pour la prédiction de survenue d'une infection associée aux soins dans les 4 jours ou dans les 7 jours suivant le prélèvement d'échantillon.**

| **Intervalle de temps entre le prélèvement de l'échantillon et l'éventuelle survenue de première infection associée aux soins** | **AUC (IC)** |
|---|---|
| 4 jours | 0,642 (0,542-0,742) |
| 7 jours | 0,657 (0,567-0,746) |

Ainsi, les résultats obtenus montrent que la mesure de l'expression de CX3CR1 seul permet de prédire la survenue d'infection(s) associée(s) aux soins dans les 15 jours à partir de l'agression immuno-inflammatoire, dans les 4 jours suivant le prélèvement de l'échantillon ou dans les 7 jours suivant le prélèvement de l'échantillon.

### Exemple 2 : La mesure de l'expression de CX3CR1 et d'un autre gène, permet d'améliorer la performance prédictive du risque de survenue d'une infection associée aux soins

### Matériels et Méthodes

Le Matériels et Méthodes est identique à celui de l'Exemple 1, à ceci près que 1) l'étape de préamplification du cDNA a été réalisée avec une autre référence de master mix PreAmp (Ref. PN100-5875 C1, Fluidigm) pour certains gènes (BTLA et IL15, ainsi que les gènes de référence qui ont été mesurés avec les 2 références) et suivie d'une étape supplémentaire de traitement par exonuclease I (ref. PN100-5875 C1, Fluidigm), et que de plus pour ces gènes, l'étape d'amplification a été réalisée avec un autre type de circuits intégrés de fluidique 192.24 (Ref. PN100-7222 C1), comme recommandé par le fournisseur, et 2) qu'ici, des régressions logistiques multivariées (combinaison de la mesure de l'expression de CX3CR1 et d'un autre gène, sélectionné dans la liste constituée par : BTLA, CD3D, CD74, CD274, CTLA4, HP, ICOS, IFNG, IL1RN, IL7R, IL10, IL15, PDCD1 et S100A9) ont été effectuées.

Ainsi, l'expression des gènes d'intérêt (à l'exception de BTLA et IL15) a été mesurée en chimie TaqMan, avec une normalisation utilisant l'expression des gènes de référence également mesurée en chimie TaqMan (référence ThermoFisher, incluant deux amorces et une sonde). L'expression des gènes BTLA et IL15 a été mesurée en chimie SYBR Green, avec une normalisation utilisant l'expression des gènes de référence également mesurée en chimie SYBR Green (référence Integrated DNA Technologies, incluant deux amorces). En plus des sondes et amorces déjà présentées dans le tableau 2 (pour CX3CR1 et les gènes de référence en chimie TaqMan), les sondes et amorces supplémentaires utilisées dans cet exemple sont présentées dans le tableau 5 (pour les autres gènes d'intérêt et les gènes de référence en chimie SYBR Green).

**Tableau 5. Sondes et amorces supplémentaires utilisées pour la qPCR**

| **Biomarqueur (gène)** | **Type** | **Référence du fournisseur ou séquences correspondant aux sondes et amorces utilisées** |
|---|---|---|
| BTLA | Gène d'intérêt | Hs.PT.58.14525368 (Integrated DNA Technologies) |
| CD3D | Gène d'intérêt | Hs00174158_m1 (ThermoFisher) |
| CD74 | Gène d'intérêt | Hs00959493_g1 (ThermoFisher) |
| CD274 | Gène d'intérêt | Hs01125301_m1 (ThermoFisher) |
| CTLA4 | Gène d'intérêt | Hs00175480_m1 (ThermoFisher) |
| HP | Gène d'intérêt | Hs00605928_g1 (ThermoFisher) |
| ICOS | Gène d'intérêt | Hs04261471_m1 (ThermoFisher) |
| IFNG | Gène d'intérêt | Hs00174143_m1 (ThermoFisher) |
| IL1RN | Gène d'intérêt | Hs00893626_m1 (ThermoFisher) |
| IL7R | Gène d'intérêt | Amorce (forward) : CTCTGTCGCTCTGTTGGTC |
| | | Amorce (reverse) : TCCAGAGTCTTCTTATGATCG |
| | | Sonde:CTATCGTATGGCCCAGTCTCC |
| IL10 | Gène d'intérêt | Hs00961620_g1 (ThermoFisher) |
| IL15 | Gène d'intérêt | Hs.PT.58.21299580 (Integrated DNA Technologies) |
| PDCD1 | Gène d'intérêt | Hs01550088_m1 (ThermoFisher) |
| S100A9 | Gène d'intérêt | Hs00610058_m1 (ThermoFisher) |
| DECR1 | Gène de référence | Hs.PT.58.19871222 (Integrated DNA Technologies) |
| HPRT1 | Gène de référence | Hs.PT.58v.45621572 (Integrated DNA Technologies) |
| PPIB | Gène de référence | Hs.PT.58v.45621572 (Integrated DNA Technologies) |

### Résultats

La mesure de l'expression d'un de ces autres gènes d'intérêt, en plus de la mesure de l'expression de CX3CR1, permet d'améliorer la performance prédictive du risque de survenue d'une infection associée aux soins, que ce soit avant le jour 15 à partir de l'inclusion dans la cohorte (Tableau 6) ou dans les 4 jours ou dans les 7 jours suivant le prélèvement de l'échantillon (Tableau 7).

**Tableau 6. Performance (AUC et intervalle de confiance à 95%, IC) de la mesure de l'expression de CX3CR1, en combinaison avec un autre biomarqueur (analyse multivariée), mesurée à J3/4 ou à J5/7 à partir de l'inclusion dans la cohorte, pour la prédiction de survenue d'une infection associée aux soins avant le jour 15 à partir de l'inclusion dans la cohorte.**

| **Jour de prélèvement de l'échantillon** | **Biomarqueurs** | **AUC (IC)** |
|---|---|---|
| J3/4 | CX3CR1 + BTLA | 0,681 (0,576-0,785) |
| J3/4 | CX3CR1 + ICOS | 0,681 (0,578-0,784) |
| J3/4 | CX3CR1 + IFNG | 0,683 (0,58-0,787) |
| J3/4 | CX3CR1 + IL15 | 0,684 (0,582-0,785) |
| J3/4 | CX3CR1 + PDCD1 | 0,693 (0,587-0,799) |
| J3/4 | CX3CR1 + HP | 0,693 (0,589-0,797) |
| J3/4 | CX3CR1 + CD74 | 0,694 (0,591-0,797) |
| J3/4 | CX3CR1 + IL10 | 0,725 (0,625-0,825) |
| J3/4 | CX3CR1 + S100A9 | 0,729 (0,615-0,844) |
| J5/7 | CX3CR1 + IFNG | 0,759 (0,655-0,862) |
| J5/7 | CX3CR1 + CD274 | 0,761 (0,66-0,863) |
| J5/7 | CX3CR1 + PDCD1 | 0,763 (0,667-0,86) |
| J5/7 | CX3CR1 + CTLA4 | 0,778 (0,674-0,883) |
| J5/7 | CX3CR1 + HP | 0,784 (0,69-0,878) |
| J5/7 | CX3CR1 + CD74 | 0,8 (0,708-0,892) |
| J5/7 | CX3CR1 + ICOS | 0,802 (0,707-0,897) |
| J5/7 | CX3CR1 + CD3D | 0,802 (0,708-0,896) |
| J5/7 | CX3CR1 + BTLA | 0,803 (0,701-0,905) |
| J5/7 | CX3CR1 + S100A9 | 0,803 (0,702-0,904) |
| J5/7 | CX3CR1 + IL7R | 0,803 (0,718-0,888) |
| J5/7 | CX3CR1 + IL1RN | 0,819 (0,726-0,911) |
| J5/7 | CX3CR1 + IL10 | 0,852 (0,775-0,928) |

**Tableau 7. Performance (AUC et intervalle de confiance à 95%, IC) de la mesure de l'expression de CX3CR1, en combinaison avec un autre biomarqueur (analyse multivariée), pour la prédiction de survenue d'une infection associée aux soins dans les 4 jours ou dans les 7 jours suivant le prélèvement d'échantillon.**

| **Intervalle de temps entre le prélèvement de l'échantillon et l'éventuelle survenue de première infection associée aux soins** | **Biomarqueurs** | **AUC (IC)** |
|---|---|---|
| 4 jours | CX3CR1 + CD3D | 0,648 (0,549-0,747) |
| 4 jours | CX3CR1 + IL15 | 0,653 (0,545-0,761) |
| 4 jours | CX3CR1 + ICOS | 0,654 (0,555-0,752) |
| 4 jours | CX3CR1 + S100A9 | 0,661 (0,563-0,759) |
| 4 jours | CX3CR1 + CD74 | 0,663 (0,566-0,761) |
| 4 jours | CX3CR1 + IFNG | 0,671 (0,572-0,771) |
| 4 jours | CX3CR1 + BTLA | 0,679 (0,582-0,776) |
| 4 jours | CX3CR1 + HP | 0,704 (0,61-0,798) |
| 7 jours | CX3CR1 + CD274 | 0,664 (0,573-0,754) |
| 7 jours | CX3CR1 + CD3D | 0,667 (0,579-0,756) |
| 7 jours | CX3CR1 + IL15 | 0,668 (0,573-0,762) |
| 7 jours | CX3CR1 + IL10 | 0,668 (0,576-0,76) |
| 7 jours | CX3CR1 + IL7R | 0,668 (0,579-0,756) |
| 7 jours | CX3CR1 + IL1RN | 0,671 (0,581-0,762) |
| 7 jours | CX3CR1 + CTLA4 | 0,671 (0,582-0,759) |
| 7 jours | CX3CR1 + IFNG | 0,676 (0,586-0,765) |
| 7 jours | CX3CR1 + ICOS | 0,686 (0,599-0,773) |
| 7 jours | CX3CR1 + BTLA | 0,692 (0,605-0,779) |
| 7 jours | CX3CR1 + CD74 | 0,694 (0,608-0,78) |
| 7 jours | CX3CR1 + S100A9 | 0,699 (0,614-0,785) |
| 7 jours | CX3CR1 + HP | 0,707 (0,622-0,792) |

## Revendications

1. Procédé *in vitro* ou *ex vivo* pour déterminer le risque de survenue d'une infection nosocomiale chez un patient au sein d'un établissement de santé, comprenant une étape de mesure de l'expression de CX3CR1, dans un échantillon biologique dudit patient.

2. Procédé selon la revendication 1, **caractérisé en ce que** le patient est un patient au sein d'un hôpital, de préférence au sein du service des urgences, du service de réanimation, en unité de soins intensifs ou en unité de soins continus, de préférence encore un patient en état septique, un patient atteint de brûlures, un patient atteint de traumatismes, ou un patient opéré par chirurgie.

3. Procédé selon l'une des revendications 1 ou 2, **caractérisé en ce qu'**il permet de déterminer le risque de survenue d'une infection nosocomiale chez le patient dans les 15 jours à partir de l'agression immuno-inflammatoire et/ou dans les 7 jours suivant le jour où le prélèvement de l'échantillon biologique a été effectué.

4. Procédé selon l'une des revendications 1 à 3, **caractérisé en ce que** l'échantillon biologique est un échantillon de sang.

5. Procédé selon l'une des revendications 1 à 4, **caractérisé en ce que** l'échantillon biologique est un échantillon de sang total.

6. Procédé selon l'une des revendications 1 à 5, **caractérisé en ce qu'**il comprend en outre une étape de mesure, dans l'échantillon biologique du patient, de l'expression d'un autre gène, sélectionné dans la liste constituée par : ADGRE3, BTLA, CD3D, CD74, CD274, CTLA4, HP, ICOS, IFNG, IL1RN, IL6, IL7R, IL10, IL15, MDC1, PDCD1, S100A9, TDRD9 et ZAP70.

7. Procédé selon l'une des revendications 1 à 6, **caractérisé en ce que** l'expression est mesurée au niveau ARNm ou protéique.

8. Procédé selon l'une des revendications 1 à 7, **caractérisé en ce que** l'expression est mesurée au niveau ARNm.

9. Procédé selon l'une des revendications 1 à 8 **caractérisé en ce que** l'expression est mesurée par RT-PCR, de préférence par RT-qPCR.

10. Procédé selon l'une des revendications 1 à 8, **caractérisé en ce que** l'expression est mesurée par séquençage.

11. Procédé selon l'une des revendications 1 à 8, **caractérisé en ce que** l'expression est mesurée par hybridation.

12. Procédé selon l'une des revendications 9 à 11, **caractérisé en ce que** l'expression est normalisée par rapport à l'expression d'un ou plusieurs gènes de ménage.

13. Utilisation de moyens d'amplification et/ou de moyens de détection de l'expression de CX3CR1 pour déterminer le risque de survenue d'une infection nosocomiale chez un patient.

## Patentansprüche

1. *In-vitro-* oder Ex-vivo-Verfahren zur Bestimmung des Risikos des Auftretens einer nosokomialen Infektion bei einem Patienten in einer Gesundheitseinrichtung, umfassend einen Schritt der Messung der Expression von CX3CR1 in einer biologischen Probe des Patienten.

2. Verfahren nach Anspruch 1, **dadurch gekennzeichnet, dass** es sich bei dem Patienten um einen Patienten in einem Krankenhaus, bevorzugt in einer Notfallstation, Reanimationsstation, Intensivstation oder Dauer-Pflegestation, weiter bevorzugt einen Patienten mit einer Sepsis, einen Patienten mit Verbrennungen, einen Patienten mit Verletzungen oder einen mittels Chirurgie operierten Patienten handelt.

3. Verfahren nach einem der Ansprüche 1 oder 2, **dadurch gekennzeichnet, dass** es die Bestimmung des Risikos des Auftretens einer nosokomialen Infektion bei dem Patienten innerhalb von 15 Tagen ab Ausbruch der immuninflammatorischen Störung und/oder innerhalb von 7 Tagen nach dem Tag, an dem die Entnahme der biologischen Probe durchgeführt wurde, ermöglicht.

4. Verfahren nach einem der Ansprüche 1 bis 3, **dadurch gekennzeichnet, dass** die biologische Probe eine Blutprobe ist.

5. Verfahren nach einem der Ansprüche 1 bis 4, **dadurch gekennzeichnet, dass** die biologische Probe eine Vollblutprobe ist.

6. Verfahren nach einem der Ansprüche 1 bis 5, **dadurch gekennzeichnet, dass** es ferner einen Schritt der Messung der Expression eines anderen Gens in der biologischen Probe des Patienten umfasst, das ausgewählt ist aus der Liste bestehend aus: ADGRE3, BTLA, CD3D, CD74, CD274, CTLA4, HP, ICOS, IFNG, IL1RN, IL6, IL7R, IL10, IL15, MDC1, PDCD1, S100A9, TDRD9 und ZAP70.

7. Verfahren nach einem der Ansprüche 1 bis 6, **dadurch gekennzeichnet, dass** die Expression auf mRNA- oder Proteinebene gemessen wird.

8. Verfahren nach einem der Ansprüche 1 bis 7, **dadurch gekennzeichnet, dass** die Expression auf mRNA-Ebene gemessen wird.

9. Verfahren nach einem der Ansprüche 1 bis 8, **dadurch gekennzeichnet, dass** die Expression mittels RT-PCR, bevorzugt mittels RT-qPCR, gemessen wird.

10. Verfahren nach einem der Ansprüche 1 bis 8, **dadurch gekennzeichnet, dass** die Expression mittels Sequenzierung gemessen wird.

11. Verfahren nach einem der Ansprüche 1 bis 8, **dadurch gekennzeichnet, dass** die Expression mittels Hybridisierung gemessen wird.

12. Verfahren nach einem der Ansprüche 9 bis 11, **dadurch gekennzeichnet, dass** die Expression auf die Expression eines oder mehrerer Haushaltsgene normalisiert wird.

13. Verwendung von Mitteln zur Amplifikation und/oder Mitteln zum Nachweisen der Expression von CX3CR1 zur Bestimmung des Risikos des Auftretens einer nosokomialen Infektion bei einem Patienten.

## Claims

1. An *in vitro* or ex *vivo* method for determining the risk of occurrence of a nosocomial infection in a patient within a healthcare facility, comprising a step of measuring the expression of CX3CR1, in a biological sample from said patient.

2. The method according to claim 1, **characterized in that** the patient is a patient within a hospital, preferably within the emergency unit, the resuscitation unit, the intensive care unit or in an on-going care unit, more preferably a patient with sepsis, a burn patient, a trauma patient, or a surgical patient.

3. The method according to any one of claims 1 or 2, **characterized in that** it allows determining the risk of occurrence of a nosocomial infection in the patient within 15 days from the immuno-inflammatory attack and/or within 7 days following the day when the biological sample collection has been performed.

4. The method according to any of claims 1 to 3, **characterized in that** the biological sample is a blood sample.

5. The method according to any of claims 1 to 4, **characterized in that** the biological sample is a whole blood sample.

6. The method according to any of claims 1 to 5, **characterized in that** it further comprises a step of measuring, in the biological sample of the patient, the expression of another gene, selected from the list consisting of: ADGRE3, BTLA, CD3D, CD74, CD274, CTLA4, HP, ICOS, IFNG, IL1RN, IL6, IL7R, IL10, IL15, MDC1, PDCD1, S100A9, TDRD9 and ZAP70.

7. The method according to any of claims 1 to 6, **characterized in that** the expression is measured at the mRNA or protein level.

8. The method according to any of claims 1 to 7, **characterized in that** the expression is measured at the mRNA level.

9. The method according to any of claims 1 to 8, **characterized in that** the expression is measured by RT-PCR, preferably by RT-qPCR.

10. The method according to any of claims 1 to 8, **characterized in that** the expression is measured by sequencing.

11. The method according to any of claims 1 to 8, **characterized in that** the expression is measured by hybridization.

12. The method according to any of claims 9 to 11, **characterized in that** the expression is normalized with respect to the expression of one or several housekeeping genes.

13. A use:
- of means for amplifying and/or means for detecting the expression of CX3CR1, or
- of a kit comprising such amplification and/or detection means,
to determine the risk of occurrence of a nosocomial infection in a patient.
